# EUROPEAN PATENT SPECIFICATION

(11) **EP 2 568 872 B1**
(45) Date of publication and mention of the grant of the patent: **06.09.2017**
(21) Application number: 11719945.5
(22) Date of filing: 03.05.2011
(51) Int. Cl.: A61B 17/34, A61B 17/00, A61B 1/313

(54) **BILIARY ACCESS SHEATH**
ZUGANGSSCHLEUSE FÜR DIE GALLE
GAINE D'ACCÈS BILIAIRE

(30) Priority: 11.05.2010 US 333335 P
(43) Date of publication of application: 20.03.2013
(73) Proprietor: Cook Medical Technologies LLC, Bloomington, IN 47404 (US)
(72) Inventor: MCHUGO, Vincent, Ireland (IE)
(74) Representative: Garratt, Peter Douglas
(86) International application number: PCT/US2011/034929
(87) International publication number: WO 2011/143003

(56) References cited:
- EP-A1- 1 964 509
- WO-A1-99/00156
- WO-A1-2010/088509
- WO-A2-2009/140594
- US-A1- 2003 078 473
- US-A1- 2006 253 123

## Description

The invention relates generally to minimally invasive surgical device accessories. More particularly, the invention pertains to a device for improving peroral gastrointestinal endoscopy access.

Intraductal endoscopes have an increasingly important role in the diagnosis and nonsurgical treatment of biliary and pancreatic diseases. US2003/078473, over which claim 1 is characterised, describes a catheter system for examination of biliary and pancreatic ducts. WO2009/140594 describes a medical access device for accessing a bodily opening. Early attempts to inspect the biliary and pancreatic ducts endoscopically have been hampered by technical limitations of the scopes. More recently, the development of fine-caliber flexible scopes known as fiber optic miniscopes has obviated many of these problems and has provided a valuable new tool for a growing number of indications. These miniature endoscopes can be used intraoperatively, during endoscopic retrograde cholangiopancreatography (ERCP, commonly performed perorally), and percutaneous transhepatic cholangiography (PTC).

Peroral cholangioscopy is usually performed by two experienced endoscopists using a "mother-baby", scope system, in which a thin fiberscope is inserted into the working channel of a large therapeutic endoscope (e.g., a duodenoscope). Smaller and more durable miniscopes allow for an accessory channel of their own. This accessory channel of the miniscopes permits sampling for histological and cytological examination and the insertion of catheters for dye or probes for laser or lithotripsy. Miniscopes such as cholangioscopes can also be used for pancreatoscopy.

The mother-baby scope technique can be expensive with regard to personnel and equipment: two endoscopists plus assistants, two image processors (one for each camera), expensive fiber optics in the baby scope that can often be damaged during standard manipulation with resulting image degradation, etc. The standard 1.2 mm working channel of fiber optic baby scopes limits diagnostic and therapeutic options. It is therefore desirable to provide an endoscope configured to function as a cholangioscope by being dimensioned to be navigable through hepatic and pancreatic ducts. Such scopes are currently available, but they encounter problems of efficient introduction to a patient's biliary duct in a procedure that provides high quality images (e.g., superior to fiber optics imaging) at a desirable procedure cost. These problems include the difficulty (or impossibility) of navigating a larger fiber optic baby scope having a greater than 1.2 mm working channel through a mother scope (e.g., duodenoscope), out its side-facing distal accessory channel end past and manipulated by the elevator, and then into a patient's biliary duct. If one is to introduce a small scope (along the size of a "baby scope" or smaller) into the biliary ducts or other patient body structure without a primary (e.g., "mother") scope, it is necessary to provide some type of "navigating track" because the smaller scopes are not sufficiently rigid/ robust to be directed/ navigated independently and directly through the esophagus, stomach, and duodenum to, for example, the common biliary duct.

Accordingly, techniques are being developed to conduct direct peroral cholangioscopy (POC). Direct POC requires only a single endoscopist working with a single image processor, using a CMOS or CCD (rather than - and with image quality superior to - fiber optic) camera system that provides a 2 mm (rather than 1.2 mm) accessory channel, and that can be used with existing scopes, image processors, and monitors. One example of such improved technology is disclosed in "Overtube-balloon-assisted direct peroral cholangioscopy by using an ultra-slim upper endoscope" (Choi, et al.; Gastrointestinal Endoscopy, 69(4):935-40; April 2009), where an over-tube with a balloon of the type used for double-balloon enteroscopy was directed into the duodenum adjacent the Ampulla of Vater with an ultra-slim scope supported in the lumen of the over-tube, whereafter the scope was directed into the previously-dilated bile duct.

In addition, after an ultra-slim scope is directed/navigated into a bile duct (whether previously dilated or not), there is a risk of it inadvertently being withdrawn during manipulation-particularly after the wire guide or other device used to guide it into the biliary tree has been withdrawn (e.g. to free up the working channel).

It would be advantageous to provide materials for efficient introduction of an ultra-slim scope suitable for cholangioscopy and pancreatoscopy in conjunction with use of a standard-sized endoscope (e.g., duodenoscope or other side-viewing or end-viewing peroral endoscopic devices, whether providing optical or computerized visualization capacity). Such materials and devices preferably will be provided without significant loss of procedural efficiency, without limiting the equipment and/or procedure to a mother-baby scope configuration, and also providing for easier, more efficient navigation into the bile duct or other locations. Such devices should also promote retention of an ufitra-slim scope in the biliary tree during a procedure.

An access sheath, preferably a biliary access sheath may be useful for introduction of an ultra-slim endoscope and/or otherwise providing access to the biliary tree of a patient. In accordance with the present invention, there is provided an access sheath as claimed in claim 1. The sheath may be a biliary access sheath and includes an elongate proximal tube portion having a fixed outer diameter and permanently attached to a distal tube portion having an outer diameter that may be constricted and expanded. The distal portion may be configured as a self-expanding tube similar to a self-expanding stent construction, being constrained during introduction into a proximal portion of the biliary tree, and released to anchor the distal sheath portion therein.

### BRIEF DESCRIPTION OF THE DRAWINGS

FIG. 1 shows a biliary access sheath;
FIG. 2 shows a partial longitudinal section of the sheath of FIG. 1;
FIG. 2A shows an external perspective view of a biliary access sheath including a flared distal self-expanding tube portion;
FIG. 3 shows a longitudinal section view of the sheath of FIG. 1 in a pre-deployment, unexpanded state;
FIG. 3A shows a longitudinal section view of the sheath of FIG. 1 in a deployed, expanded state;
FIGS. 4-4A show an externally-constrained embodiment of a biliary access sheath;
FIG. 4B shows another externally-constrained embodiment of a biliary access sheath; and
FIGS. 5A-5C show a method for introducing an intra-ductal endoscope using the biliary access sheath of FIG. 1.

### DETAILED DESCRIPTION

### DEFINITIONS

Ultra-slim endoscopes, as that term is used herein, refer to endoscopes having an outer diameter of about 6.0 mm or less (including less than 5.0 mm), and particularly includes an ultra-slim intraductal endoscope using optical, digital (e.g., CMOS, CCD), or ultrasound imaging. The terms "distal" and "proximal" are to be understood with their standard usages, referring to the direction away from and the direction toward the handle/ user end of a tool or device, respectively (i.e., the term "distal" means the direction or portion of the device that is farthest from the physician or other person operating the tool or device and the term "proximal" means the portion of the device that is nearest to that physician or other person).

Embodiments are described with reference to the drawings in which like elements are generally referred to by like numerals. The relationship and functioning of the various elements of the embodiments may better be understood by reference to the following detailed description. However, embodiments are not limited to those illustrated in the drawings. It should be understood that the drawings are not necessarily to scale, and in certain instances details may have been omitted that are not necessary for an understanding of embodiments of the present invention, such as - for example -conventional fabrication and assembly.

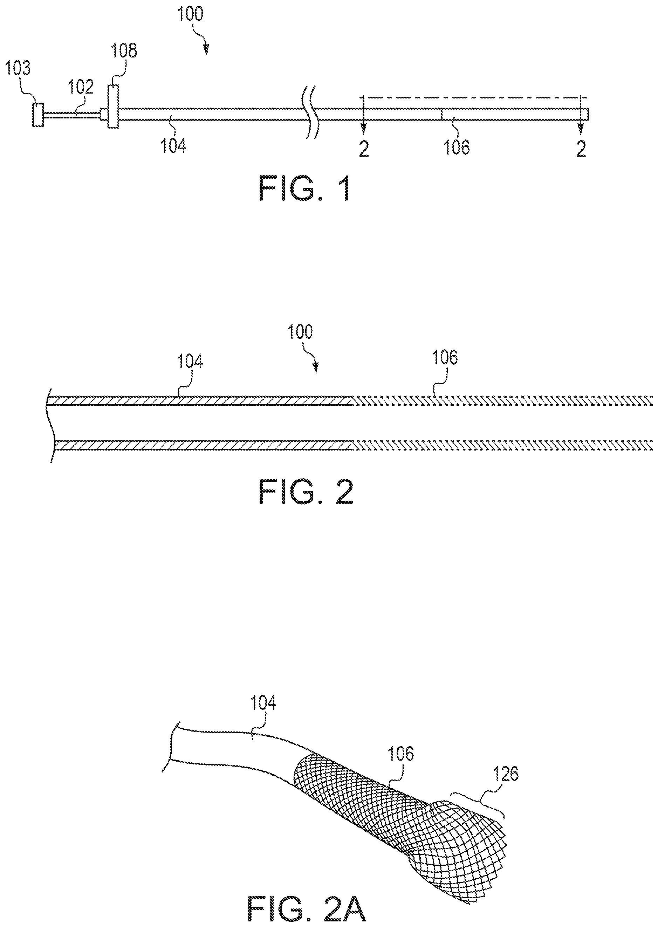

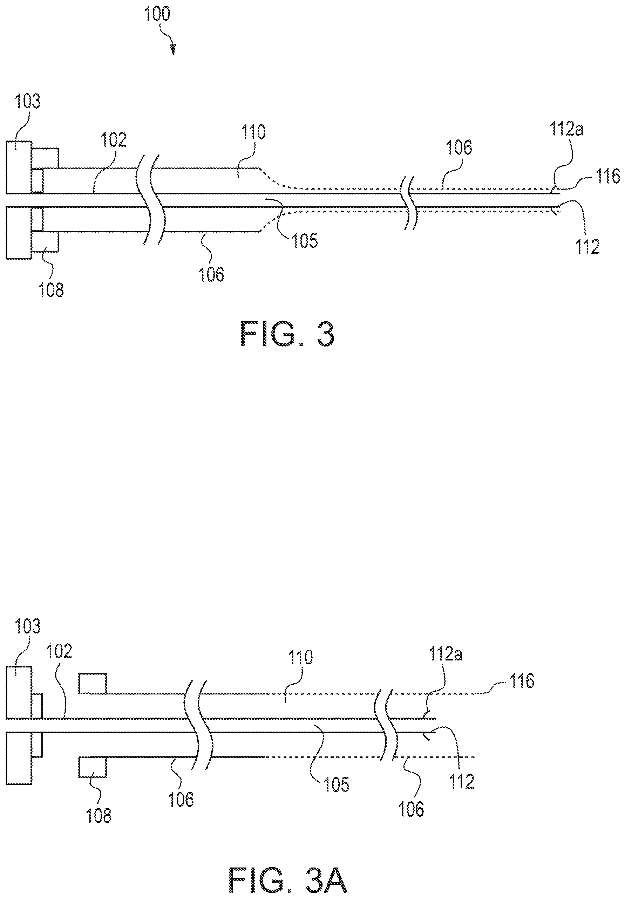

One example of a biliary access sheath 100 is described with reference to FIG. 1. The sheath 100 has an elongate tubular body including a proximal body portion 104 that is permanently affixed to a distal body portion 106. A longitudinal lumen (not visible in FIG. 1, see - for example - lumen 110 in FIG. 3) extends continuously through the proximal and distal body portions 104, 106. The sheath 100 preferably will be configured with sufficient length and flexibility for peroral, trans-esophageal navigation of the distal body portion to a biliary duct of a patient. A pusher member 102 extends through the length of the sheath lumen. A proximal pusher member handle 103 preferably is configured with a removable connection to a proximal end of the proximal body portion 104 (not shown as engaged in FIG. 1), which may be configured as a sheath handle 108.

The proximal body portion 104 preferably is configured as a tubular catheter body that has a substantially static/ constant outer diameter, which may have some radial flexibility, but which maintains a generally consistent outer diameter, although it may be radially deformable and/or bendable in the manner of other tubular bodies such as catheters. The distal body portion 106 most preferably is configured to include an expandable/collapsible construction that is biased into an expanded state so as to comprise a self-expanding tube. The tube 106 is configured for passage from a patient's duodenal lumen into the biliary duct when in a non-expanded (that is, radially low-profile) state. The tube 106 is also configured to engage the biliary duct when in an expanded state. When in the non-expanded state, the tube 106 includes an outer diameter that is less than the outer diameter of the proximal body portion 104.

The proximal body portion 104 may be constructed with nylon, PET, PTFE, polyurethane, or other tubing, which may be reinforced with stainless steel coil or other metallic tubing. Or metallic tubing may be used, preferably with lubricious coating on its inner and outer surfaces. The proximal body portion 104 preferably is constructed to provide trackability and pushability that will facilitate passage over a wire guide and/or through a working channel of a peroral endoscope (such as, for example, a side-viewing duodenoscope).

The distal body portion, configured as a self-expanding tube 106 is shown diagrammatically in longitudinal section in FIG. 2 (along line 2-2 of FIG. 1). The self-expanding tube may be constructed in a manner substantially similar or identical to that of self-expanding stents. For example, the tube 106 may be constructed as a woven double-helical NiTi wire tube, which is preset into a radially-expanded configuration, but which can be constrained in a radially low-profile non-expanded state. In such an access sheath some or all of the NiTi wire may be coated with, for example, a low friction or hydrophilic coating. Shape memory materials other than NiTi may be used including polymeric materials. FIG. 2A shows an external perspective view of one exemplary construction of a distal body portion 106, shown in an expanded state with a flared distal region 126 that is configured to enhance its ability to anchor within a patient biliary duct. In its non-expanded state, the distal sheath portion 106 preferably will have an outer diameter that is less than the outer diameter of the proximal sheath portion 104, such as is shown - for example - in FIG. 3.

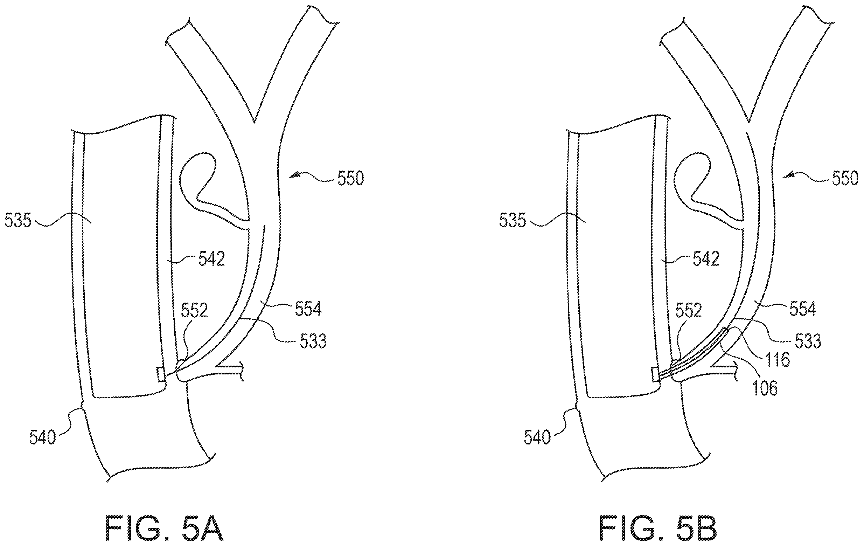

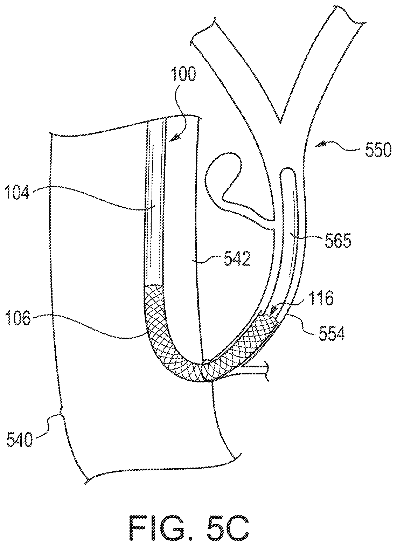

The type of construction used in metallic and/or polymeric self-expanding stents such as, for example, the Evolution® stent (Cook Endoscopy, Winston-Salem, N.C.) or Zilver® biliary stent (Cook Inc., Bloomington, Ind.) may be used or adapted to form the distal sheath portion. Other constructions that may be used or adapted for use within embodiments of the device disclosed herein include, for example, those disclosed and/or discussed in U.S. Pat. No. 5,507,771 to Gianturco; U.S. Pat. No. 5,968,088 to Hansen et al.; U.S. Pat. No. 7,582,110 to Case et al.; U.S. Pat. No. 7,625,399 to Case et al.; and U.S. Pat. No. 7,658,759 to Case et al.; as well as U.S. Pat. Publ. No. 2005/0125050 to Carter et al. The construction of the distal body portion 106 may also include a pre-set curve (also well-known in the art relative to stents and similar constructs) that is configured to support the longitudinal lumen in an open and generally unrestricted manner when that body portion 106 is occupying the transition/curve from the duodenal lumen 542 to the biliary duct 554 as shown, for example, in FIG. 5.

A low friction or hydrophilic coating on at least some components of the distal body portion 106 may be configured as a sleeve forming a substantially fluid-patent lumen for at least some length of that portion. Such a sleeve may be configured as being discontinuous to allow fluid passage through one or more regions of the distal body portion 106. For example, it may be advantageous to allow for flushing of the longitudinal body lumen with a saline solution, and one or more apertures or other open regions in the distal body portion 106 (and/or in the proximal body portion 104) may facilitate the ability to direct fluid through the device 100. Preferred coatings for the proximal and distal body portions 104, 106 preferably will include a lubricious profile that will ease passage of the device 100 relative to other components (e.g. wire guide, endoscope) and vice versa. One or more markers configured to be echogenic and/or radio-opaque may be included on the distal body portion 106 and/or proximal body portion 104 to assist in location and navigation of the device 100 within a patient body (e.g., by ultrasound and/or fluoroscopic visualization).

FIG. 3 shows a more detailed view of the biliary access sheath 100. The pusher handle 103 is releasably attached to the sheath handle 108 (e.g., by a friction-fit, threaded connection, Luer-type ½ or %-turn connection, bayonet connection, or other suitable connection of those types well known in the art for easy connection and removal of tubular or other components from each other). The pusher body 102 extends through the longitudinal sheath lumen 110 and is shown as including a pusher lumen 105 that preferably is sized to accommodate at least passage of a wire guide, and preferably is sized to accommodate passage of a low-profile anchor balloon catheter (such as, for example, a Cook Fusion® Dilation Balloon (Cook Endoscopy, Winston-Salem, NC)). The distal end 112 of the pusher body 102 is engaged with the distal-most end 116 of the distal body portion 106.

The distal body portion 106 is configured as a self-expanding (i.e., preset into a radially-expanded configuration) woven double-helical NiTi wire tube. As is known with this type of construction (e.g., in self-expanding stents) radial compression/constraint corresponds to longitudinal lengthening of the tube 105, similar to that commonly known and observed with "Chinese finger cuffs." Conversely, foreshortening of the tube corresponds with its radial expansion. This phenomenon is utilized in the present device 100 such that when the pusher handle 103 is engaged with the sheath handle 108 and the distal pusher end 112 is releasably engaged with the distal-most end 116 of the distal body portion 106, that distal body portion 106 is stretched lengthwise in a manner reducing its outer diameter to the non-expanded state.

It should be appreciated that numerous means for this engagement to effect releasable connection between an internal pusher/ restraining member and a self-expanding tube that are known and/or that are still being developed in the art may be used within the scope of the present invention. As one example, a retention wire may be used to effect releasable connection between the distal pusher end 112 and the distal-most body portion end 116 as described in U.S. Pat. Publ. No. 2009/0030497 to Metcalf et al. This and other release structures may also be configured to be re-captured and/or otherwise re-activated to re-constrain the distal tube portion 106 to a lower profile. In the illustrated embodiment of FIG. 3, simply hook-like protrusions 112a extend from the pusher 102 to engage the distal-most tube end 116. Releasing the distal pusher end 112 from the distal-most end 116 of the distal body portion 106 will allow that distal tube 106 to deploy/expand to the configuration shown in FIG. 3A. In most embodiments, this release/deployment will correspond to releasing the proximal pusher handle 103 from the sheath handle 108 and proximally retracting the pusher 102 relative to the distal sheath body portion 106.

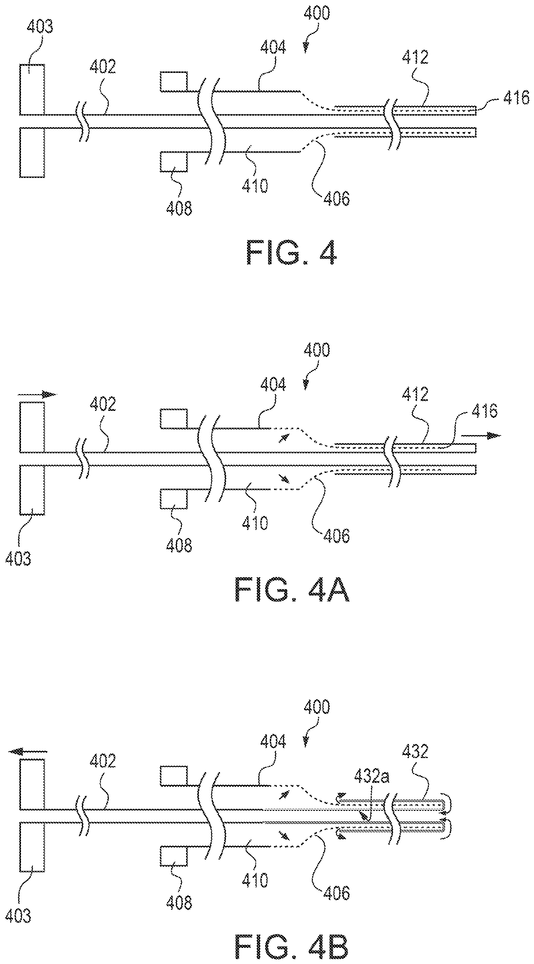

In one exemplary embodiment, the proximal body portion may be constructed of nylon tube reinforced with stainless steel coil, about 90 cm in length. The distal body portion may be constructed as a woven double-helical NiTi wire tube with a lubricious hydrophilic coating forming a flexible barrier sleeve for much of its length of about 10 cm (when in an expanded state). In an unexpanded state the outer diameter of the distal body portion may be about 4 mm, and about 9 mm in its expanded state. The inner diameter of the proximal body portion (and the distal body portion when in its radially expanded state) will be at least about 6 mm. An embodiment of a biliary access sheath 400, using an external rather than an internal constraint for a self-expanding tube portion 406 is described with reference to FIG. 4. The sheath 400 has an elongate tubular body including a proximal body portion 404 that is permanently affixed to a distal body portion 406. A longitudinal lumen 410 extends continuously through the proximal and distal body portions 404, 406. The sheath 400 preferably will be configured with sufficient length and flexibility for peroral, trans-esophageal navigation of the distal body portion to a biliary duct of a patient. A pusher member 402 extends through the length of the sheath lumen. A proximal pusher member handle 403 is disposed proximal of the sheath handle 408.

The distal body portion 406 includes an expandable/collapsible construction that is biased into an expanded state so as to comprise a self-expanding tube. The tube 406 is configured for passage from a patient's duodenal lumen into the biliary duct when in a constrained, non-expanded (that is, radially low-profile) state. The tube 406 is also configured to engage and anchor the device 400 into the biliary duct when in an expanded state.

The distal end of the pusher 402 is configured as an overlying pusher constraint sleeve 412 that extends distally past the distal-most tube end 416 and then back proximally to at least partially cover and thereby constrain the self-expanding tube 406 by a releasable connection. When in the constrained non-expanded state, the tube 406 and overlying pusher constraint sleeve 412 include a total outer diameter that preferably is less than the outer diameter of the proximal body portion 404. The constraint sleeve 412 is configured to maintain the self-expanding tube portion 406 in a low-profile non-expanded state.

FIG. 4A shows how a semi-rigid constraint sleeve 412 may be advanced distally toward and then past the distal-most tube end 416 (and/or be held in place while the tube is drawn proximally) to deploy the self-expanding tube end 406. This deployment is effected as the tube end 406 expands itself upon removal from constraint. FIG. 4B shows an alternative constraint element 432 constructed as a flexible bi-layer evertible sleeve. With reference to FIG. 4B (and particularly the motion arrows therein), it will be appreciated that proximal retraction of the inward-facing layer 432a will evert the sleeve 432, thereby shortening the constraining portion thereof that overlies the tube 406 and freeing the tube to deploy/ expand radially. In either embodiment, releasing the distal pusher end sleeve 412/432 from the distal-most end 416 of the distal body portion 406 will allow that distal tube end 406 to deploy/expand to the configuration shown, for example, in FIG.2 or 2A.

The biliary access sheath described herein may have many uses, but particularly be useful in a method for accessing a biliary tree with an ultra-slim endoscope (e.g., for visualization and/or for conducting a surgical, diagnostic, and/or other procedure). Methods are described with reference to elements shown in FIGS. 1, 2, and 5A-5B (although other embodiments, such as-for example-those shown in FIGS. 4A and 4B may be used). Other methods are described in U.S. Pat. App. Ser. No. 61/256,773 to Dillon et al., filed Oct. 30, 2009, and from which WO 2011/053500 claims priority. In one such method, ERCP may be performed to visualize the biliary tree 550 of a patient (not to scale: shown much larger than typical relative to the duodenum for illustrative purposes only). A peroral endoscope 535 (shown in FIG. 5A as a duodenoscope) may be directed through the esophagus and stomach into the duodenum 540 of a patient, adjacent the sphincter of Oddi 552, opening into the biliary duct 554. Whether or not ERCP has been performed, the biliary access sheath 100 may be directed along a wire guide 533 or a catheter of an anchoring balloon configured to function like a distally-anchored wire guide, the distal end of which is disposed in or through the patient's biliary duct via a working channel of the endoscope 535. In certain methods, the endoscope 535 may be removed before introducing the biliary access sheath 100.

As shown in FIG. 5B, the distal-most end 116 of the distal sheath portion 106 (engaged with the distal-most pusher end 112), in its non-expanded state is directed into the biliary duct 554 via the sphincter of Oddi 552, which may have been cannulated via sphincterotomy. A sufficient length to provide anchoring-including accounting for foreshortening upon deployment-preferably will be directed into the biliary duct 554. This directing step may be done along the anchoring catheter/wire guide 533, after which the endoscope 535 may be removed.
Then, the proximal pusher handle 103 will be disconnected from the proximal sheath handle 108 and the pusher 102 withdrawn proximally, allowing the self-expanding tube forming the distal sheath portion to expand radially - most preferably with sufficient force to anchor into the biliary duct 554 as shown in FIG. 5C. In certain anchoring structures such as flared tube portions, wings, higher-friction surfaces (e.g., uncoated wire portions), barbs or the like may be included on the distal tube portion 106. However, it will be preferable that such structures are configured to minimize the possibility of damage to the biliary duct. For example, a retracting means may be provided for re-contraction/constraint of the distal tube member 106 to minimize the likelihood of damaging the biliary duct 554 when the device 100 is removed. Various such means for collapsing, restraining, and/or otherwise reducing the profile/ outer diameter of self-expanding structures such as self-expanding stents are known and are being developed within the art, including embodiments constructed not to exhibit foreshortening upon constriction and expansion.

As shown in FIG. 5C, after the biliary access sheath 100 is in place, an ultra-slim scope 565 (such as, for example, an intra-ductal endoscope) may be directed through the lumen 110 and up into the biliary tree 550. The ultra-slim scope 565 may be directed along the wire guide 533, which then may be removed to free up the working channel of the scope 565. Thereafter, at least one of a surgical procedure or diagnostic procedure may be conducted via the ultra-slim scope, which may be advanced to extend well beyond the distal-most end 116 of the distal sheath portion 106. The biliary access sheath 100 may enhance the efficiency of such procedures in several ways. For example, whether or not the distal sheath portion 106 is pre-curved, the curvature taken when it is anchored in the biliary duct 554 will generally prevent
proximal/retrograde movement of the ultra-slim endoscope 565 disposed therethrough and will help to stabilize it during procedures. In addition, unlike procedures that use a wire guide or anchoring balloon disposed through the working channel of the ultra-slim scope 565 to keep it anchored/oriented in the biliary tree 550, the access sheath 100 will allow that working channel to be free for other uses. The access sheath 100 may also lessen the possibility of the ultra-slim scope 565 getting twisted or kinked as it is being directed through the stomach lumen or duodenal lumen 542.

Those of skill in the art will appreciate that embodiments not expressly illustrated herein may be practiced within the scope of the present invention as defined in the claims. Features described herein for different embodiments may be combined with each other and/or with currently-known or future-developed technologies while remaining within the scope of the claims presented here (e.g., use of a sheath for urological, gynecological, respiratory, or other body lumen applications). It is therefore intended that the foregoing detailed description be regarded as illustrative rather than limiting. And, it should be understood that the following claims are intended to define the scope of this invention. Furthermore, the advantages described above are not necessarily the only advantages of the invention, and it is not necessarily expected that all of the described advantages will be achieved with every embodiment of the invention.

## Claims

1. An access sheath (400) comprising:
an elongate tubular body including a proximal body portion (404) and a distal body portion (406), and comprising a longitudinal lumen (410) extending through a length of the elongate tubular body;
the proximal body portion (404) comprising a first outer diameter;
the distal body portion (406) comprising an expandable/collapsible construction that is biased into an expanded state so as to comprise a self-expanding tube, configured for passage into a patient duct when in a non-expanded state, and further configured to anchor into a patient duct when in an expanded state,
**characterized in that** the access sheath (400) further comprises an elongate pusher member (402) extending through the longitudinal lumen (410) and connected releasably to the distal body portion (406);
wherein a distal length of the pusher member is configured as a constraining sleeve (412, 432) that extends around and constrains an outer diameter of at least a portion of the self-expanding tube in a manner retaining that portion in a non-expanded state; and
wherein the distal length of the pusher member may be moved to deploy the constrained self-expanding tube by releasing the self-expanding tube to assume an expanded state.

2. The access sheath (400) of claim 1, wherein the pusher member (402) is also releasably connected to the proximal body portion (406).

3. The access sheath (400) of claim 1, wherein the elongate pusher member (402) extending through the longitudinal lumen (410) is connected releasably to the distal body portion (406) that is configured as the self-expanding tube.

4. The access sheath (400) of claim 3, wherein the non-expanded state corresponds to the pusher member (402) being connected to the distal body portion (406), and the expanded state corresponds to the pusher member not being connected to the distal body portion.

5. The access sheath (400) of claim 3, wherein the pusher member (402) comprises a removable connection with the proximal body portion (406).

6. The access sheath (400) of claim 1, wherein the distal body portion (406) comprises a woven double-helical shape memory material.

7. The access sheath (400) of claim 6, wherein the proximal body portion (404) comprises nylon, PET, PTFE, or polyurethane tubing and stainless steel coil configured to reinforce the proximal body portion.

8. The access sheath (400) of claim 1, wherein an inner diameter of the proximal body portion (404) is at least about 6 mm.

9. The access sheath (400) of claim 1, wherein an inner diameter of the proximal body portion (404) comprises a lubricious surface.

10. The access sheath (400) of claim 1, wherein the distal body portion (406) comprises at least one marker that is configured to be echogenic, radio-opaque, or a combination thereof, said marker disposed and configured to promote visualization of the distal body portion during navigation.

11. The access sheath (400) of claim 1, wherein a distalmost length of the distal body portion (406) is configured as a self-expanding tube,
wherein the elongate pusher member (402) comprises a distal-end constraining sheath (432) configured to externally engage and constrain the self-expanding distalmost length of the distal body portion (406) to a smaller outer diameter than the proximal body potion (404),
wherein the smaller outer diameter is configured for passage into a patient duct, and
wherein distal movement of the pusher and constraining sheath is configured to release and allow expansion of the self-expanding distalmost length of the distal body portion.

12. An access sheath (400) as claimed in any one of the preceding claims, wherein the distal body portion is permanently affixed to the proximal body portion;
the longitudinal lumen extends continuously through the proximal and distal body portions; and
wherein the distal body portion has a contracted outer diameter that is less than the first outer diameter of the proximal body portion, and an expanded outer diameter that is greater than the contracted outer diameter, where the contracted outer diameter is configured and dimensioned for passage into a patient duct, and where the expanded outer diameter is configured and dimensioned to longitudinally anchor at least a length of the distal body portion by radial contact within a patient duct.

13. The access sheath of any preceding claim, wherein:
the access sheath is a biliary access sheath,
the proximal body portion (404) and the distal body portion (406) are together configured with sufficient length and flexibility for trans-esophageal navigation of the distal body portion to a biliary duct of a patient, and
the longitudinal lumen (410) is configured to allow passage of a low-profile gastric endoscope therethrough when the distal body is in the expanded state.

14. The biliary access sheath (400) of claim 13, wherein the self-expanding tube is configured with a pre-set curvature configured to transition from a duodenal lumen space to a biliary duct when it is an expanded state.

## Patentansprüche

1. Zugangsschleuse (400), umfassend:
einen länglichen röhrenförmigen Körper, der einen proximalen Körperabschnitt (404) und einen distalen Körperabschnitt (406) aufweist und ein Längslumen (410) umfasst, das sich durch eine Länge des länglichen röhrenförmigen Körpers erstreckt,
wobei der proximale Körperabschnitt (404) einen ersten äußeren Durchmesser umfasst,
wobei der distale Körperabschnitt (406) eine expandierbare/zusammenklappbare Konstruktion umfasst, die in einen expandierten Zustand vorgespannt ist, so dass sie eine selbstexpandierende Röhre umfasst, die dazu konfiguriert ist, in einen Gang eines Patienten zu gehen, wenn sie in einem nicht expandierten Zustand ist, und ferner dazu konfiguriert ist, sich in einem Gang eines Patienten zu verankern, wenn sie in einem expandierten Zustand ist,
**dadurch gekennzeichnet, dass** die Zugangsschleuse (400) ferner ein längliches Schieberglied (402) umfasst, das sich durch das Längslumen (410) erstreckt und freigebbar mit dem distalen Körperabschnitt (406) verbunden ist,
wobei eine distale Länge des Schieberglieds als eine Beschränkungshülse (412, 432) konfiguriert ist, die sich um einen äußeren Durchmesser mindestens eines Abschnitts der selbstexpandierenden Röhre herum erstreckt und diesen so beschränkt, dass der Abschnitt in einem nicht expandierten Zustand gehalten wird, und
wobei die distale Länge des Schieberglieds zum Ablegen der beschränkten selbstexpandierenden Röhre bewegt werden kann, indem die selbstexpandierende Röhre freigegeben wird, um einen expandierten Zustand einzunehmen.

2. Zugangsschleuse (400) nach Anspruch 1, wobei das Schieberglied (402) ebenfalls freigebbar mit dem proximalen Körperabschnitt (406) verbunden ist.

3. Zugangsschleuse (400) nach Anspruch 1, wobei das sich durch das Längslumen (410) erstreckende längliche Schieberglied (402) freigebbar mit dem distalen Körperabschnitt (406) verbunden ist, der als die selbstexpandierende Röhre konfiguriert ist.

4. Zugangsschleuse (400) nach Anspruch 3, wobei der nicht expandierte Zustand dem entspricht, dass das Schieberglied (402) mit dem distalen Körperabschnitt (406) verbunden ist, und der expandierte Zustand dem entspricht, dass das Schieberglied nicht mit dem distalen Körperabschnitt verbunden ist.

5. Zugangsschleuse (400) nach Anspruch 3, wobei das Schieberglied (402) eine entfernbare Verbindung mit dem proximalen Körperabschnitt (406) umfasst.

6. Zugangsschleuse (400) nach Anspruch 1, wobei der distale Körperabschnitt (406) ein doppelspiralförmiges Formgedächtnismaterialgewebe umfasst.

7. Zugangsschleuse (400) nach Anspruch 6, wobei der proximale Körperabschnitt (404) Nylon, PET, PTFE oder einen Polyurethanschlauch und eine Edelstahlspirale umfasst, die zur Verstärkung des proximalen Körperabschnitts konfiguriert sind.

8. Zugangsschleuse (400) nach Anspruch 1, wobei ein innerer Durchmesser des proximalen Körperabschnitts (404) mindestens ungefähr 6 mm beträgt.

9. Zugangsschleuse (400) nach Anspruch 1, wobei ein innerer Durchmesser des proximalen Körperabschnitts (404) eine gleitfähige Oberfläche umfasst.

10. Zugangsschleuse (400) nach Anspruch 1, wobei der distale Körperabschnitt (406) mindestens einen Marker umfasst, der dazu konfiguriert ist, echogen, röntgendicht oder eine Kombination davon zu sein, wobei der Marker so angeordnet und konfiguriert ist, dass er eine Visualisierung des distalen Körperabschnitts während des Navigierens fördert.

11. Zugangsschleuse (400) nach Anspruch 1, wobei eine distalste Länge des distalen Körperabschnitts (406) als eine selbstexpandierende Röhre konfiguriert ist,
wobei das längliche Schieberglied (402) eine Beschränkungshülse (432) am distalen Ende umfasst, die dazu konfiguriert ist, die selbstexpandierende distalste Länge des distalen Körperabschnitts (406) extern in Eingriff zu nehmen und auf einen kleineren äußeren Durchmesser als den proximalen Körperabschnitt (404) zu beschränken,
wobei der kleinere äußere Durchmesser für den Durchgang in einen Gang eines Patienten konfiguriert ist und
wobei eine distale Bewegung des Schiebers und der Beschränkungshülse dazu konfiguriert ist, die selbstexpandierende distalste Länge des distalen Körperabschnitts freizugeben und deren Expansion zu gestatten.

12. Zugangsschleuse (400) nach einem der vorhergehenden Ansprüche, wobei der distale Körperabschnitt permanent an dem proximalen Körperabschnitt befestigt ist,
das Längslumen sich durchgehend durch den proximalen und den distalen Körperabschnitt erstreckt und wobei der distale Körperabschnitt einen zusammengezogenen äußeren Durchmesser hat, der kleiner als der erste äußere Durchmesser des proximalen Körperabschnitts ist, und einen expandierten äußeren Durchmesser, der größer als der zusammengezogene äußere Durchmesser ist, wobei der zusammengezogene äußere Durchmesser für den Durchgang in einen Gang eines Patienten konfiguriert und bemessen ist, und wobei der expandierte äußere Durchmesser dazu konfiguriert und bemessen ist, mindestens eine Länge des distalen Körperabschnitts durch radialen Kontakt mit einem Gang eines Patienten in Längsrichtung zu verankern.

13. Zugangsschleuse nach einem der vorhergehenden Ansprüche, wobei
die Zugangsschleuse eine Zugangsschleuse für die Galle ist,
der proximale Körperabschnitt (404) und der distale Körperabschnitt (406) zusammen mit hinreichender Länge und Flexibilität für die trans-ösophageale Navigation des distalen Körperabschnitts zu einem Gallengang eines Patienten konfiguriert sind, und
das Längslumen (410) dazu konfiguriert ist, den Durchgang eines flach ausgeführten Magenendoskops dort hindurch zu gestatten, wenn der distale Körper im expandierten Zustand ist.

14. Zugangsschleuse (400) nach Anspruch 13, wobei die selbstexpandierende Röhre mit einer voreingestellten Krümmung konfiguriert ist, die dazu konfiguriert ist, aus einem Zwölffingerdarmlumenraum zu einem Gallengang überzugehen, wenn sie in einem expandierten Zustand ist.

## Revendications

1. Gaine d'accès (400) comprenant :
un corps tubulaire allongé comprenant une partie de corps proximale (404) et une partie de corps distale (406), et comprenant une lumière longitudinale (410) s'étendant à travers une longueur du corps tubulaire allongé ;
la partie de corps proximale (404) comprenant un premier diamètre extérieur ;
la partie de corps distale (406) comprenant une structure dilatable/compressible qui est sollicitée vers un état dilaté de façon à constituer un tube auto-dilatable, configuré pour le passage dans une voie d'un patient lorsqu'il se trouve dans l'état non dilaté, et configuré en outre pour s'ancrer dans une voie d'un patient lorsqu'il se trouve dans l'état dilaté,
**caractérisée en ce que** la gaine d'accès (400) comprend en outre un élément formant poussoir (402) allongé s'étendant à travers la lumière longitudinale (410) et raccordé de manière libérable à la partie de corps distale (406) ;
une longueur distale de l'élément formant poussoir étant configurée sous forme d'une gaine de retenue (412, 432) qui s'étend autour d'un diamètre extérieur d'au moins une partie du tube auto-dilatable et la retient de façon à maintenir cette partie dans un état non dilaté ; et
la longueur distale de l'élément formant poussoir pouvant être déplacée de façon à déployer le tube auto-dilatable retenu par la libération du tube auto-dilatable de sorte qu'il passe à l'état dilaté.

2. Gaine d'accès (400) selon la revendication 1, dans laquelle l'élément formant poussoir (402) est également raccordé de manière libérable à la partie de corps proximale (406).

3. Gaine d'accès (400) selon la revendication 1, dans laquelle l'élément formant poussoir (402) allongé s'étendant à travers la lumière longitudinale (410) est raccordé de manière libérable à la partie de corps distale (406) qui est configurée sous la forme du tube auto-dilatable.

4. Gaine d'accès (400) selon la revendication 3, dans laquelle l'état non-dilaté correspond au fait que l'élément formant poussoir (402) est raccordé à la partie de corps distale (406), et l'état dilaté correspond au fait que l'élément formant poussoir n'est pas raccordé à la partie de corps distale.

5. Gaine d'accès (400) selon la revendication 3, dans laquelle l'élément formant poussoir (402) comprend un raccordement amovible avec la partie de corps proximale (406).

6. Gaine d'accès (400) selon la revendication 1, dans laquelle la partie de corps distale (406) comprend un matériau à mémoire de forme tissé à double hélice.

7. Gaine d'accès (400) selon la revendication 6, dans laquelle la partie de corps proximale (404) comprend du nylon, du PET, du PTFE ou un tube en polyuréthane et une bobine en acier inoxydable configurée pour renforcer la partie de corps proximale.

8. Gaine d'accès (400) selon la revendication 1, dans laquelle un diamètre intérieur de la partie de corps proximale (404) est d'au moins environ 6 mm.

9. Gaine d'accès (400) selon la revendication 1, dans laquelle un diamètre intérieur de la partie de corps proximale (404) comprend une surface lubrifiée.

10. Gaine d'accès (400) selon la revendication 1, dans laquelle la partie de corps distale (406) comprend au moins un marqueur qui est configuré pour être échogène, radio-opaque ou une combinaison de ces propriétés, ledit marqueur étant disposé et configuré pour favoriser la visualisation de la partie de corps distale lors de la navigation.

11. Gaine d'accès (400) selon la revendication 1, dans laquelle une longueur la plus distale de la partie de corps distale (406) est configurée sous la forme d'un tube auto-dilatable,
dans laquelle l'élément formant poussoir (402) allongé comprend une gaine de retenue d'extrémité distale (432) configurée pour venir en prise extérieurement avec la longueur la plus distale auto-dilatable de la partie de corps distale (406) et la retenir de sorte qu'elle présente un diamètre extérieur inférieur à celui de la partie de corps proximale (404),
dans laquelle le diamètre extérieur inférieur est configuré en vue du passage dans une voie d'un patient, et
dans laquelle le déplacement dans la direction distale du poussoir et de la gaine de retenue est configuré pour libérer la longueur la plus distale auto-dilatable de la partie de corps distale et permettre sa dilatation.

12. Gaine d'accès (400) selon l'une quelconque des revendications précédentes, dans laquelle la partie de corps distale est fixée à demeure à la partie de corps proximale ;
la lumière longitudinale s'étend de façon continue à travers les parties de corps proximale et distale ; et
dans laquelle la partie de corps distale présente un diamètre extérieur contracté, qui est inférieur au premier diamètre extérieur de la partie de corps proximale, et un diamètre extérieur dilaté, qui est supérieur au diamètre extérieur contracté, le diamètre extérieur contracté étant configuré et dimensionné en vue du passage dans une voie d'un patient, et le diamètre extérieur dilaté étant configuré et dimensionné afin d'ancrer longitudinalement au moins une longueur de la partie de corps distale par contact radial à l'intérieur d'une voie d'un patient.

13. Gaine d'accès selon l'une quelconque des revendications précédentes, dans laquelle :
la gaine d'accès est une gaine d'accès biliaire,
la partie de corps proximale (404) et la partie de corps distale (406) sont configurées pour présenter conjointement une longueur et une souplesse suffisantes pour une navigation transoesophagienne de la partie de corps distale jusqu'à une voie biliaire d'un patient, et
la lumière longitudinale (410) est configurée pour permettre le passage d'un endoscope gastrique à profil bas à travers elle lorsque le corps distal se trouve dans l'état dilaté.

14. Gaine d'accès biliaire (400) selon la revendication 13, dans laquelle le tube auto-dilatable est configuré de façon à présenter une courbure préétablie configurée pour former une transition entre un espace de lumière duodénale et une voie biliaire lorsqu'il se trouve dans l'état dilaté.
